# EUROPÄISCHE PATENTANMELDUNG

(11) **EP 1 245 221 A1**
(43) Veröffentlichungstag der Anmeldung: **02.10.2002**
(21) Anmeldenummer: 02005259.3
(22) Anmeldetag: 11.03.2002
(51) Int. Cl.: A61K 7/46, A61L 9/14

(54) **Benetzungsadditiv**

(30) Priorität: 23.03.2001 DE 10114510
(71) Anmelder: HAARMANN & REIMER GMBH, 37603 Holzminden (DE)
(72) Erfinder: Mansfeld, Gerd, 37632 Eschershausen (DE); Harzke, Falk, 37619 Bodenwerder (DE); Eilers, Jörg, 37619 Hehlen (DE); Müller, Dirk, 37586 Dassel (DE)
(74) Vertreter: Zobel, Manfred, Dr.

(57) **Zusammenfassung**

Die Erfindung betrifft die Verwendung von Fettsäureniederalkylestem zur Einstellung der Viskosität bei Parfümkompositionen sowie Mischungen enthaltend Fettsäureniederalkylester und Parfümkompositionen oder Duftstoffe und deren Verwendung.

## Beschreibung

Die Erfindung betrifft die Verwendung von Fettsäureniederalkylestern zur Einstellung der Viskosität bei Parfümkompositionen sowie Mischungen enthaltend Fettsäureniederalkylester und Parfümkompositionen oder Duftstoffe und deren Verwendung.

Parfümkompositionen oder Parfümöle bestehen aus einer Vielzahl organischer Verbindungen. Diese Verbindungen, auch Duftstoffe oder Parfümeinsatzstoffe genannt, weisen produktspezifische Eigenschaften auf. Hierzu zählen bespielsweise Geruch, Farbe, Dichte, optische Drehung, Refraktionsindex, Viskosität, Polarität, Oberflächenspannung oder Flammpunkt. Bei der Entwicklung neuer Parfümkompositionen durch den Parfümeur spielen die physikalischen und chemischen Produkteigenschaften der Einsatzstoffe normalerweise eine untergeordnete Rolle. Der Parfümeur erstellt seine Kreationen anhand der geruchlichen Eigenschaften der Parfümeinsatzstoffe. Es gibt jedoch Anwendungen, bei denen die produktspezifischen Eigenschaften von Parfümkompositionen eine nicht unerhebliche Rolle spielen. Dies ist beispielweise der Fall, wenn Parfümkompositionen oder Duftstoffe über Flüssigverdampfer-Systeme auf Basis der Piezotechnologie oder über automatische Dosiereinrichtungen ausgebracht werden und eine gegenseitige Kontamination der Duftstoffe oder der Parfümkompositionen an den dicht nebeneinander liegenden Dosieröffnungen der Dosierköpfe verhindert werden soll.

Ein Vergleich von Wasser und Parfümkompositionen möge diese Problematik verdeutlichen. Dicht nebeneinanderliegende Wassertropfen, die z.B. auf einer Glasplatte aufgetragen werden (im Vergleich: kleine Tropfen (ca. 10 - 20 mg) im Abstand von 3-5 mm zueinander), neigen insgesamt deutlich weniger zu einem Verlaufen der Tropfen, als die Parfümkompositionen unter gleichen Versuchsbedingungen. Die Parfümkompositionen zeigen in ihrem Benetzungs-, Fließ- und Kontaminationsverhalten teilweise deutliche Mängel.

Es bestand daher die Aufgabe, Duftstoffe und / oder Parfümkompositionen derart zu modifizieren, dass eine gegenseitige Kontamination durch Verlaufen oder Fließen der jeweiligen Flüssigkeitstropfen weitestgehend verhindert wird. Die Modifikation soll dabei mit geruchsneutralen oder geruchslosen Mitteln erfolgen, da diese Mittel den sensorischen Eindruck der Duftstoffe oder der Parfümkompositionen nicht verfälschen dürfen.

Es wurde nun überraschenderweise gefunden, dass durch Zusatz von Fettsäureniederalkylestern mit insgesamt 15 bis 20 Kohlenstoffatomen zu Parfümölen, Parfümkompositionen oder Duftstoffen das Benetzungs,- Fließ- und Kontaminationsverhalten der Mischung enthaltend Fettsäureniederalkylester und Parfümöle, Parfümkompositionen oder Duftstoffe deutlich verbessert werden kann.

Gegenstand der vorliegenden Erfindung ist daher die Verwendung von Fettsäureniederalkylestern zur Einstellung der Viskosität von Mischungen enthaltend Parfümkompositionen oder Duftstoffe, dadurch gekennzeichnet, dass die Fettsäureniederalkylester insgesamt 15 bis 20 Kohlenstoffatome enthalten. Ein weiterer Gegenstand der vorliegenden Erfindung sind Mischungen mit einer Viskosität von 6 bis 25 mPas enthaltend Parfümkompositionen oder Duftstoffe, dadurch gekennzeichnet, dass diese Fettsäureniederalkylester insgesamt 15 bis 20 Kohlenstoffatome enthalten, sowie die Verwendung dieser Mischungen zur Auftragung auf harte Oberflächen und zur piezoelektrisch gesteuerten Duftaustragung.

Unter Niederalkyl sind im vorliegenden Falle Methyl, Ethyl, n-Propyl, Isopropyl, n-Butyl, Isobutyl, sek-Butyl und tert-Butyl zu verstehen, bevorzugte Niederalkylreste sind Ethyl und Isopropyl, insbesondere bevorzugt ist Isopropyl.

Die erfindungsgemäßen Fettsäureniederalkylester sind an sich bekannt und kommerziell erhältlich.

Bevorzugt sind Fettsäureniederalkylester mit insgesamt 16 bis 19 Kohlenstoffatomen.

Besonders vorteilhafte Fettsäureniederalkylester sind Isopropylpalmitat, Ethylpalmitat, Ethylmyristat, Isopropylmyristat und Ethylstearat, insbesondere bevorzugt ist Isopropylmyristat (1-Methylethyltetradecanoat).

Weiterhin wurde nun gefunden, dass ein optimales Benetzungs,- Fließ- und Kontaminationsverhalten über die Viskosität der Mischung enthaltend Fettsäureniederalkylester und Parfümkompositionen, Parfümöl oder Duftstoff eingestellt werden kann.

Die Viskosität der Mischung enthaltend Fettsäureniederalkylester und Parfümkompositionen oder Duftstoff liegt bevorzugt im Bereich von 6 bis 25 mPas, insbesondere bevorzugt im Bereich von 9 bis 15 mPas.

Die Viskositäten der in der Riechstoffindustrie üblichen Parfümkompositionen liegen typischerweise in einem Bereich von 1 bis 110 mPas.

Die Viskositäten wurden mit einem Fallkörperviskosimeter (Kugel-Fall-Methode) bestimmt. Die Messungen erfolgten mit dem Gerät "MicroVisko 2" der Firma Haake bei 24,5°C unter Verwendung einer 3 mm Kugel.

Der Anteil der Fettsäureniederalkylester in der Mischung enthaltend Fettsäureniederalkylester und Parfümkompositionen oder Duftstoff liegt bevorzugt im Bereich von 1 bis 80 Gew.-%, insbesondere bevorzugt im Bereich von 10 bis 50 Gew.-%.

Weiterhin können die in der Parfümölindustrie üblicherweise eingesetzten Lösungsmittel wie beispielsweise Diethylphthalat (1,2-Benzoldicarbonsäure-diethylester), Dipropylenglycol (1,1'-Oxybis-1-propanol), Triethylcitrat (2-Hydroxy-1,2,3-propantricarbonsäure-triethylester) im Bereich von 1 bis 50 % zugesetzt werden.

Die erfindungsgemäßen Mischungen sind insbesondere geeignet für den Einsatz zur piezoelektrisch gesteuerten Duftaustragung sowie zur Aufbringung auf harte Oberflächen.

Hauptbestandteile der harten Oberflächen können beispielsweise Glas, Keramik, Metall, Porzellan, Holz, natürliche oder synthetische Polymere oder ähnliche Materialien sein. In bevorzugten Ausführungsformen werden die erfindungsgemäßen Mischungen als Reinigungsmittel eingesetzt, insbesondere als Haushaltsreiniger. Es seien beispielsweise Mittel zur Reinigung von Fensterglas, Parkett, Laminatfußboden oder Linoleum genannt.

Das folgende Beispiel erläutert die Erfindung.

### Beispiel 1:

In Tabelle 1 ist die Modifizierung der Viskosität mit Fettsäureniederalkylestern, speziell Isopropylmiyristat, von Mischungen enthaltend Parfümöle dargelegt.

Es wurde ein enger Viskositätsbereich eingestellt, der besonders zur piezoelektrisch gesteuerten Duftaustragung geeignet ist.

**Tabelle 1:**

| Viskositätsmodifizierung von Mischungen enthaltend Parfümöle mit Isopropylmyristat (Angaben in Gew.-%). | | | | |
|---|---|---|---|---|
| Viskosität Parfümöl | Parfümöl | Isopropyl-myristat | Dipropylen-glycol | Eingestellte Viskosität |
| [mPa*s] | [%] | [%] | [%] | [mPa*s] |
| 2,017 | 42 | 10 | 48 | 9,750 |
| 5,094 | 60 | 10 | 30 | 9,872 |
| 5,498 | 50 | 10 | 30 | 9,970 |
| 8,748 | 70 | 10 | 20 | 10,830 |
| 11,780 | 85 | 15 | 0 | 9,815 |
| 13,200 | 80 | 20 | 0 | 9,900 |
| 14,000 | 75 | 25 | 0 | 9,840 |
| 17,200 | 60 | 40 | 0 | 10,080 |
| 19,680 | 61 | 39 | 0 | 9,881 |
| 21,600 | 60 | 40 | 0 | 10,890 |
| 25,850 | 55 | 45 | 0 | 9,760 |
| 27,250 | 51 | 49 | 0 | 9,643 |
| 31,880 | 53 | 47 | 0 | 9,866 |
| 34,760 | 40 | 60 | 0 | 9,980 |
| 39,830 | 40 | 60 | 0 | 9,728 |
| 54,680 | 40 | 20 | 0 | 10,600 |
| 76,260 | 37 | 63 | 0 | 9,750 |
| 106,100 | 28 | 72 | 0 | 9,649 |

Die zu 100 % fehlenden prozentualen Anteile waren andere übliche geruchslose Lösungsmittel oder andere Fettsäureniederalkylester.

## Patentansprüche

1. Verwendung von Fettsäureniederalkylestern zur Einstellung der Viskosität von Mischungen enthaltend Parfümkompositionen oder Duftstoffe, **dadurch gekennzeichnet, dass** die Fettsäureniederalkylester insgesamt 15 bis 20 Kohlenstoffatome enthalten.

2. Verwendung nach Anspruch 1, **dadurch gekennzeichnet, dass** die Mischung einen Gehalt an Fettsäureniederalkylester von 1 bis 80 Gew.-% aufweist.

3. Verwendung nach mindestens einem der Ansprüche 1 bis 2, **dadurch gekennzeichnet, dass** die Mischung eine Viskosität von 6 bis 25 mPas aufweist.

4. Verwendung nach mindestens einem der Ansprüche 1 bis 3, **dadurch gekennzeichnet, dass** der Fettsäureniederalkylester Isopropylmyristat ist.

5. Mischungen mit einer Viskosität von 6 bis 25 mPas enthaltend Parfümkompositionen oder Duftstoffe, **dadurch gekennzeichnet, dass** diese Fettsäureniederalkylester insgesamt 15 bis 20 Kohlenstoffatome enthalten

6. Mischungen enthaltend Parfümkompositionen oder Duftstoffe nach Anspruch 5, **dadurch gekennzeichnet, dass** diese einen Gehalt an Fettsäureniederalkylestern von 1 bis 80 Gew.-% aufweisen.

7. Verwendung der Mischungen gemäß den Ansprüchen 5 oder 6 zur piezoelektrisch gesteuerten Duftaustragung.

8. Verwendung der Mischungen gemäß den Ansprüchen 5 oder 6 zur Aufbringung auf harte Oberflächen.
